# EUROPÄISCHE PATENTANMELDUNG

(11) **EP 1 162 260 A2**
(43) Veröffentlichungstag der Anmeldung: **12.12.2001**
(21) Anmeldenummer: 01110422.1
(22) Anmeldetag: 27.04.2001
(51) Int. Cl.: C12M 1/107

(54) **Kofermentationsanlage**

(30) Priorität: 06.05.2000 DE 10022056
(71) Anmelder: U.T.S. Umwelt-Technik-Süd GmbH, 84419 Obertaufkirchen (DE)
(72) Erfinder: Bürger, Adam, 84405 Grüntegernbach (DE)
(74) Vertreter: Neubauer, Hans-Jürgen, Dipl.-Phys.

(57) **Zusammenfassung**

Die Erfindung betrifft eine Kofermentationsanlage (1) für die gemeinsame Vergärung eines Basissubstrats (5) und organischer Rückstände als Kosubstrate zur Erzeugung von Biogas. Die Kofermentationsanlage (1) umfasst einen Fermenter als Reaktor (2) sowie eine Basissubstratzuführung (4) und eine Kosubstratzuführung zum Reaktor (2). Ferner ist eine Vorbehandlungsvorrichtung (3) zur Zerkleinerung und zur Erwärmung von Kosubstraten vorgesehen. Dem Reaktor (2) ist ferner ein Gasspeicher und eine Gasverwertungsvorrichtung, insbesondere als Brenner oder Blockheizkraftwerk nachgeschaltet. Erfindungsgemäß weist die Vorbehandlungsvorrichtung (3) ein Vorbehandlungsrohr (10) auf, das an einem Ende für eine Kosubstrateinspeisung (18) hergerichtet ist und dessen anderes Ende über einen Kosubstratauslass (19) mit dem Reaktor (2) direkt oder indirekt verbunden ist. Das Vorbehandlungsrohr (10) ist zumindest in Teilbereichen doppelwandig ausgeführt und das dadurch geschaffene Zwischenvolumen (13) an eine Heizwasserversorgung angeschlossen, so dass bei eingebrachtem Heizwasser (14) das Innenvolumen des Vorbehandlungsrohrs (10) erwärmbar und darin eingebrachte Kosubstrate thermisch vorbehandelbar, insbesondere thermisch hygienisierbar sind. Im Vorbehandlungsrohr (10) ist eine sich axial erstreckende, antreibbare Fördereinrichtung (16) als Transport- sowie als Misch- und/oder Zerkleinerungseinrichtung angebracht.

## Beschreibung

Die Erfindung betrifft eine Kofermentationsanlage nach dem Oberbegriff des Anspruchs 1.

Kofermentationsanlagen werden insbesondere im landwirtschaftlichen Bereich für die gemeinsame Vergärung eines Basissubstrats, vorzugsweise von Gülle, und organischer Rückstände aus Gewerbe, Industrie und Kommunen als Kosubstrate zur Erzeugung von Biogas in einem Nassgärverfahren eingesetzt.

Allgemein und schematisch ist eine Kofermentationsanlage in einem Positionspapier des Kuratoriums für Technik und Bauwesen in der Landwirtschaft (KTBL-Sonderveröffentlichung 014, 1997) beschrieben. Wesentliche Komponenten einer solchen Kofermentationsanlage sind ein Fermenter als Reaktor mit eingebauten Mischeinrichtungen und mit einer Basissubstratzuführung zum Reaktor. Weiter ist eine Kosubstratzuführung zum Reaktor vorzusehen mit einer Vorbehandlungseinrichtung zur Zerkleinerung, zur Mischung und zur Erwärmung der Kosubstrate. Dem Reaktor ist in allgemein bekannter Weise ein Gasspeicher für das erzeugte Biogas und eine Gasverwertungseinrichtung insbesondere als Brenner- oder als Blockheizkraftwerk nachgeschaltet. Unter Kofermentation wird allgemein die gemeinsame Vergärung von Flüssigmist oder speziell vorbehandeltem Festmist zusammen mit landwirtschaftlichen, gewerblichen, agroindustriellen oder kommunalen biogenen Abfällen zur Gewinnung von Biogas verstanden. Mit der Kofermentation soll eine verantwortbare Rückführung organischer Abfälle einschließlich der darin enthaltenen Nährstoffe ähnlich dem landwirtschaftlichen Stoffkreislauf erreicht und gleichzeitig erzeugtes Biogas energietechnisch genutzt werden. Abhängig von der Herkunft landwirtschaftlich betriebsfremder Stoffe sind besondere Maßnahmen zur Sicherstellung der seuchenhygienischen Unbedenklichkeit der Produkte und zur Verhinderung von Schadstoffanreicherungen bei der landbaulichen Ausbringung der Gärprodukte erforderlich.

Dazu ist es allgemein bekannt Kosubstrate wie Bioabfälle, Fettabscheiderinhalte, Großküchenabfälle, Schlachthofabfälle etc. vor der Einbringung in den Reaktor einer mechanischen Zerkleinerung und einer thermischen Hygienisierung zu unterziehen. Für eine thermische Hygienisierung ist es bekannt zerkleinerte Kosubstrate über die Dauer von mindestens einer Stunde einer Temperaturerhöhung von mindestens 70° Celsius auszusetzen. Dadurch werden insbesondere relevante Tierseuchenerreger sicher abgetötet, wenn seuchenhygienisch bedenkliche Stoffe verarbeitet werden.

Aufgabe der Erfindung ist es eine konstruktiv einfache Lösung für eine Vorbehandlungseinrichtung einer Kofermentationsanlage aufzuzeigen, die zudem mit wenig Aufwand kostengünstig betreibbar ist.

Diese Aufgabe wird mit den Merkmalen des Anspruchs 1 gelöst.

Gemäß Anspruch 1 weist die Vorbehandlungsvorrichtung ein Vorbehandlungsrohr auf, dessen eines Ende für eine Kosubstrateinspeisung hergerichtet ist und dessen anderes Ende über einen Kosubstratauslass direkt oder indirekt Verbindung zum Reaktorvolumen hat.

Das Vorbehandlungsrohr ist zumindest in Teilbereichen doppelwandig ausgeführt und das dadurch geschaffene Zwischenvolumen ist an eine Heizwasserversorgung angeschlossen. Durch eingebrachtes Heizwasser bestimmter Temperatur können das Innenvolumen des Vorbehandlungsrohrs und darin eingebrachte Kosubstrate erwärmt und thermisch hygienisiert werden.

Zudem ist im Vorbehandlungsrohr eine sich axial erstreckende antreibbare Fördereinrichtung als Transporteinrichtung und/oder als Misch- und/oder Zerkleinerungseinrichtung angebracht. Vorzugsweise erstreckt sich die Fördereinrichtung über die gesamte Länge des Vorbehandlungsrohres und füllt das zylindrische Innenvolumen des Vorbehandlungsrohres weitgehend aus.

Damit sind in nur einer Vorbehandlungsvorrichtung in der Art eines Vorbehandlungsrohrs je nach den Gegebenheiten und Erfordernissen eine Mischung, eine Zerkleinerung, eine thermische Hygienisierung und ein Transport der Kosubstrate möglich. Dadurch wird diese Vorrichtung kompakt und preisgünstig herstellbar bei einfach überschaubarem, und gegebenenfalls automatisiert steuerbaren Betrieb. Eine Anpassung an unterschiedliche Gegebenheiten kann insbesondere durch die Dauer und Laufrichtung gegebenenfalls mit umgesteuerten Laufrichtungen der Fördereinrichtung erfolgen. Damit lassen sich seuchenhygienische Erfordernisse bei der Verarbeitung von Kosubstraten insbesondere auch bei kleineren landwirtschaftlichen Kofermentationsanlagen einfach und kostengünstig erfüllen, so dass Kofermentationsverfahren auch für kleinere landwirtschaftliche Betriebe interessant werden mit den eingangs erwähnten positiven Effekten für die Landwirtschaft und den Umweltschutz.

Gemäß Anspruch 2 ist es vorteilhaft am Vorbehandlungsrohr zur Kosubstrateinspeisung ein ggf. beheizbares etwa vertikales Zuführrohr anzubringen, dessen unterer Bereich eine Förderverbindung zum zugeordneten Ende der Fördereinrichtung aufweist. Dies kann beispielsweise mit einer Einführschräge erfolgen oder dadurch dass das Ende der Fördereinrichtung unmittelbar in den Bereich der Zuführrohrunterseite eingreift.

Grundsätzlich kann das Kosubstrat kontinuierlich aus dem Zuführrohr durch die angetriebene Fördereinrichtung entnommen werden. Es ist jedoch auch eine chargenweise Beschickung des Vorbehandlungsrohrs möglich insbesondere durch eine Schließeinrichtung vorzugsweise als steuerbare Klappe oder steuerbarer Schieber am unteren Bereich des Zuführrohrs. Das Zuführrohr kann auch als Vorratsbehälter ausgebildet sein und je nach Bedarf und Anlieferung von Kosubstraten chargenweise befüllt werden.

Grundsätzlich kann das Vorbehandlungsrohr in jeder Position z. B. vertikal angeordnet sein wobei hier ungünstigerweise Kosubstrat durch sein Gewicht undefinierbar nach unten rutschen kann. Mit Anspruch 3 wird daher vorgeschlagen, das Vorbehandlungsrohr etwa horizontal anzuordnen, wodurch die Position des Kosubstrats im Vorbehandlungsrohr im wesentlichen durch die Einwirkung der Fördereinrichtung bestimmbar ist. Möglicherweise kann eine leichte Schrägstellung von der Kosubstrateinspeisung zum Kosubstratauslass ansteigend zweckmäßig sein, insbesondere wenn am Kosubstratauslass vorbehandeltes Kosubstrat frei aus dem Vorbehandlungsrohr durch die Fördereinrichtung ausgeschoben werden soll.

Mit den Merkmalen des Anspruchs 4 wird vorgeschlagen, dass der Kosubstratauslass des Vorbehandlungsrohres im Bereich der Basissubstratzuführung am Reaktor angeordnet ist. Vorteilhaft können damit sowohl das Basissubstrat als auch das vorbehandelte Kosubstrat an einer gemeinsamen Stelle dem Reaktor zugeführt werden. Dabei ist eine kontinuierliche Kosubstratzuführung zum Reaktor ebenso wie eine chargenweise Kosubstratzuführung möglich. Dazu wird zweckmäßig am Kosubstratauslass des Vorbehandlungsrohres eine Schließeinrichtung beispielsweise als steuerbare Klappe oder steuerbarer Schieber angebracht.

Eine einfache und bevorzugte Fördereinrichtung besteht gemäß Anspruch 5 aus einer Schneckenfördereinrichtung, wobei die Schnecke im Vorbehandlungsrohr axial angeordnet ist. Mit einem solchen Schneckenantrieb kann eine effektive und gezielte Förderung in Verbindung mit einer Mischung und Zerkleinerung von Kosubstrat durchgeführt werden.

In einer konstruktiv einfachen Lösung nach Anspruch 6 soll der Schneckenantrieb insbesondere als Elektromotor koaxial zur Schnecke außerhalb des Vorbehandlungsrohres angeordnet werden.

Die Schnecke kann hinsichtlich ihres Durchmessers, ihrer Steigung und ihres Antriebs an unterschiedliche Gegebenheiten und an die zu verarbeitenden Kosubstrate angepasst werden. Insbesondere kann nach Anspruch 7 die Schnecke umfangseitig strukturiert ausgebildet werden. Ebenso kann auch die Innenwand des Vorbehandlungsrohrs eine zugeordnete Struktur aufweisen. Mit zahnförmigen oder zackenförmigen Strukturen gegebenenfalls in Verbindung mit Schlagleisten kann eine Zerkleinerung und Mischung gefördert werden. Durch gezielte Ausnehmungen am Umfangsrand kann auch ein Verklemmschutz erreicht werden, insbesondere wenn Knochenabfälle verarbeitet werden sollen.

Für eine intensive Zerkleinerung und Mischung kann die Drehrichtung der Schnecke gegebenenfalls auch mehrmals umgesteuert werden, so dass das Kosubstrat bei drehender Schnecke lange im Vorbehandlungsrohr gehalten wird ohne dass das Kosubstrat aus dem Kosubstratauslass gefördert wird.

Alternativ zu der Schneckenfördereinrichtung wird nach Anspruch 8 eine Rohfördereinrichtung in einem vorzugsweise ringförmig umlaufenden Vorbehandlungsrohr vorgeschlagen. Die Rohrfördereinrichtung umfasst wenigstens ein umlaufendes Zugelement vorzugsweise eine Kette und/oder ein Seil, wobei im Abstand daran Förderelemente in der Art von Mitnehmerscheiben oder Mitnehmerschaufel angeordnet sind. Da hier die Mitnehmerelemente an den Innenwänden des Vorbehandlungsrohres entlang geführt sind, ergibt sich dadurch eine Reinigung des Rohrinneren. Die Zugelemente und Mitnehmerelemente können ggf. einfach beim Vorbeigang am Kosubstratauslass einfach gereinigt werden. Insbesondere können sich bei einer solchen Rohrfördereinrichtung Vorteile bei Reinigungs- und Wartungsarbeiten gegenüber einer Schneckenfördereinrichtung ergeben.

Um das Temperaturgefälle zwischen dem Heizwassermantel des Vorbehandlungsrohrs und der Umgebung für eine gute Energiebilanz möglichst gering zu halten wird mit Anspruch 9 vorgeschlagen eine Wärmedämmung an der Außenwand des Vorbehandlungsrohres anzubringen. Geeignete Wärmedämmungen sind allgemein bekannt.

Nach Anspruch 10 wird das Heizwasser mit bestimmter Temperatur in einem Heizwasserkreislauf durch das Zwischenvolumen des Vorbehandlungsrohrs gepumpt. Dabei ist sicherzustellen, dass dieser gesamte Bereich durchströmt und erhitzt wird. Maßnahmen dazu sind aus der Heiztechnik allgemein bekannt. Insbesondere sollen der Heizwasserzulauf und Heizwasserablauf versetzt angebracht sein. Gegebenenfalls können Leitsysteme durch Leitbleche und/oder im Zwischenvolumen enthaltene Heizrohre angebracht werden. Durch eine Gesamtbeheizung des Zwischenvolumens gibt vorteilhaft die gesamte Innenwand des Vorbehandlungsrohres Wärme an dort anliegende Kosubstrate ab, so dass eine effektive und für eine thermische Hygienisierung ausreichende Erhitzung des gesamten im Vorbehandlungsrohr enthaltenen Kosubstrats auf wenigstens 70° Celsius erreichbar ist.

Zweckmäßig wird nach Anspruch 11 für die Erhitzung des Heizwassers direkt oder indirekt das im Reaktor erzeugte Biogas verwendet. Dazu kann ein Heizofen mit Brenner und Wärmetauscher als separate Einheit eingesetzt werden. Auch die Abwärme eines Blockheizkraftwerks kann für die Erhitzung des Heizwassers herangezogen werden.

Die Vorbehandlungsvorrichtung kann mittels einer Steuereinrichtung nach Anspruch 12 weitgehend automatisiert werden, so dass der Betrieb der Anlage nur einen geringen Arbeitsaufwand erfordert. Mit einer solchen Steuereinrichtung können vorgebbare Zeitsteuerungen und Drehzahlsteuerungen für den Lauf der Fördereinrichtung in Verbindung mit entsprechend angepassten Öffnungs- und Schließvorgängen von Schließeinrichtungen gesteuert werden. Zudem kann eine Temperaturregelung für die Behandlungstemperatur eingesetzt werden. Steuerungen und Regelungen sind in jedem Fall so durchzuführen, dass die Verweildauer von Kosubstrat im Vorbehandlungsrohr bei einer wenigstens 70° Celsiuserhitzung wenigstens eine Stunde beträgt.

Flüssige Kosubstrate z. B. Fette, Molkereiabwasser, flüssige Lebensmittel können in Tankfahrzeugen in größeren Mengen bis zu 30 Tonnen an der Kofermentationsanlage angeliefert werden und müssen in einem Vorlagetank gelagert werden, bevor sie nach den vorstehenden Vorschlägen kontinuierlich hygienisiert werden. Bei fetthaltigen Anteilen muss der Vorlagetank beheizt werden, damit die Fettanteile immer flüssig bleiben. Mit den Merkmalen des Anspruchs 13 wird vorgeschlagen, dass zumindest ein Teilbereich des beheizbaren Vorbehandlungsrohrs im Vorlagetank angeordnet ist, so dass vom Vorbehandlungsrohr nach außen abgegebene Wärme zur Beheizung des Vorlagetanks genutzt wird, ggf. in Verbindung mit einer weiteren zusätzlichen Heizeinrichtung. Dabei soll nach Anspruch 14 zweckmäßig der Einlauf in das Zuführrohr in der Höhe eines Flüssigkeitsspiegels des Vorlagetanks liegen und der Kosubstratauslass soll dann als Überlauf in eine entsprechende Höhe nach oben geführt sein.

Anhand einer Zeichnung wird die Erfindung näher erläutert:

Es zeigen:
- Fig. 1: eine schematische Darstellung einer Kofermentationsanlage mit einer ersten Ausführungsform einer Vorbehandlungseinrichtung,
- Fig. 2: eine schematische Darstellung einer Vorbehandlungseinrichtung in einem Vorlagetank,
- Fig. 3: eine weitere Ausführungsform einer Vorbehandlungseinrichtung in einem Vorlagetank, und
- Fig. 4: einen Schnitt entlang der Linie A - A in Fig. 3.

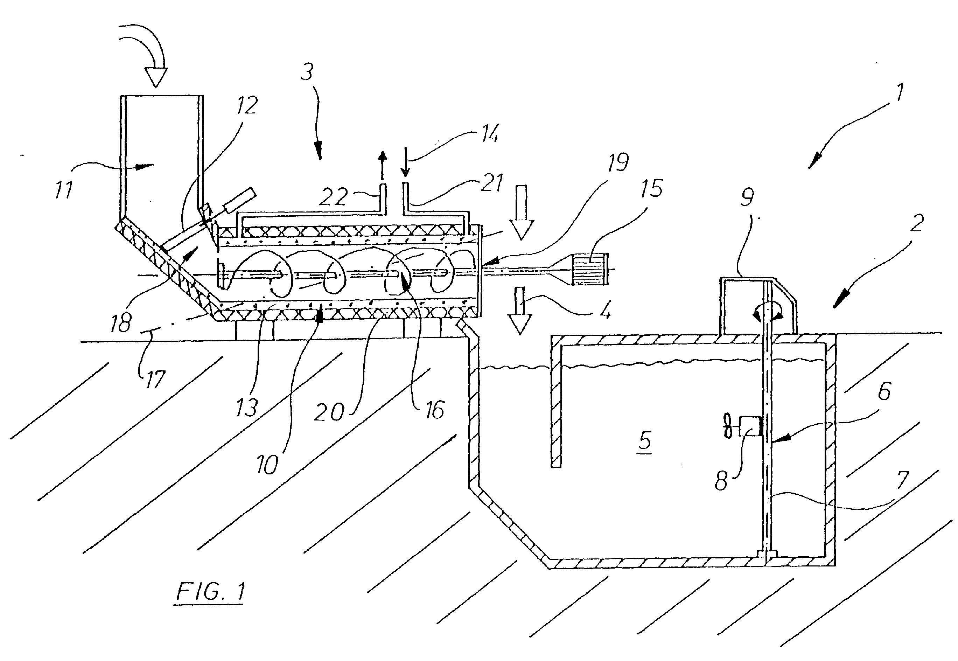

Fig. 1 zeigt schematisch eine Kofermentationsanlage 1, die einen Fermenter 2 als Reaktor sowie eine Vorbehandlungsvorrichtung 3 umfasst.

Der Fermenter 2 weist eine hier lediglich schematisch dargestellte Basissubstratzuführung 4 auf, über die z. B. Gülle als Basissubstrat 5 dem Fermenter 2 zugeführt wird.

Wie dies Fig. 1 weiter zu entnehmen ist, umfasst der Fermenter 2 ferner eine Mischeinrichtung 6, die ein im Fermenter 2 gelagertes Trägerrohr 7 umfasst, an dem eine Rühreinrichtung 8 höhenverstellbar gehalten ist. Das Trägerrohr 7 kann ebenfalls verschwenkt werden und ragt mit einem oberen Ende aus dem Fermenter 2 heraus, wobei dieses obere Ende wenigstens teilweise in einem Servicedom 9 verläuft, in den die Rühreinrichtung 8 zum Zwecke von Wartungsarbeiten angehoben werden kann.

Die Vorbehandlungsvorrichtung 3 weist ein Vorbehandlungsrohr 10 auf, an dessen hinterem Ende für eine Kosubstrateinspeisung ein trichterförmiges Zuführrohr 11 angeordnet ist. An dessen unterem Bereich ist ferner ein steuerbarer Schieber 12 für eine chargenweise Beschickung des Vorbehandlungsrohrs 10 mit hier nicht dargestelltem Kosubstrat, z. B. Speiseabfällen, vorgesehen.

Ferner ist im Vorbehandlungsrohr 10 eine sich axial erstreckende, mittels eines Antriebsmotors 15 antreibbare Transporteinrichtung als Transportschnecke 16 angeordnet, mit der z. B. bei dauerhaft geöffnetem Schieber 12 kontinuierlich Kosubstrat aus dem Zuführrohr 11 in das Vorbehandlungsrohr 10 gefördert werden kann.

Aus der Fig. 1 ist weiter ersichtlich, dass das Vorbehandlungsrohr 10 hier beispielhaft im wesentlichen horizontal ausgerichtet angeordnet ist. Alternativ kann jedoch, wie dies durch die schräggestellte Achse 17 angedeutet ist, das Vorbehandlungsrohr 10 auch mit einem leichten Anstieg von der Kosubstrateinspeisung 18 zu einem Kosubstratauslass 19 hin ausgerichtet sein. Dieser Kosubstratauslass 19 ist hier im Bereich der Basissubstratzuführung 4 am Fermenter 2 angeordnet. Am Kosubstratauslass 19 kann je nach dem ob eine kontinuierliche oder eine chargenweise Kosubstratzuführung zum Fermenter 2 vorgesehen ist, auch eine Schließeinrichtung in Form einer steuerbaren Klappe oder eines steuerbaren Schiebers vorgesehen sein, was hier allerdings nicht dargestellt ist.

Die Transportschnecke 16 ist umfangsseitig vorzugsweise strukturiert ausgebildet, wobei auch die Innenwand des Vorbehandlungsrohrs 10 eine entsprechend zugeordnete Strukturierung aufweisen kann. Ferner können Ausnehmungen am Umfangsrand der Transportschnecke 16 als Verklemmschutz vorgesehen sein, was hier allerdings ebenfalls nicht dargestellt ist.

Der Fig. 1 kann zudem weiter entnommen werden, dass an der Außenwandung des Vorbehandlungsrohres 10 eine Wärmedämmung 20 angebracht ist.

Wie dies der Fig. 1 weiter entnommen werden kann, ist das Vorbehandlungsrohr 10 doppelwandig ausgeführt und das dadurch geschaffene Zwischenvolumen 13 an eine Heizwasserversorgung angeschlossen, so dass bei eingebrachtem Heizwasser 14 das Innenvolumen des Vorbehandlungsrohrs 10 erwärmbar und die darin eingebrachten Kosubstrate thermisch hygienisierbar sind. Das Heizwasser 14 wird über einen Heizwasserzulauf 21 in das Zwischenvolumen 13 des Vorbehandlungsrohrs 10 mit einer solchen Temperatur gepumpt, dass eine thermische Hygienisierung der sich im Vorbehandlungsrohr 10 befindlichen Kosubstrate auf wenigstens 70°C erfolgt, wodurch z. B. Tierseuchenerreger sicher abgetötet werden können. Das Heizwasser 14 kann dabei über einen Heizwasserablauf 22 wieder aus dem Zwischenvolumen 13 abgezogen werden. Vorteilhaft wird dabei die Schneckendrehzahl und/oder werden die Schließeinrichtungen dabei so gesteuert, dass das Kosubstrat im Vorbehandlungsrohr 10 wenigstens eine Stunde verweilt.

Über den Kosubstratauslass 19 gelangt dann das thermisch hygienisierte Kosubstrat in den Fermenter 2.

Die Erhitzung des Heizwassers 14 erfolgt dabei vorzugsweise direkt oder indirekt mittels der Verbrennung von dem im Fermenter 2 erzeugten Biogas.

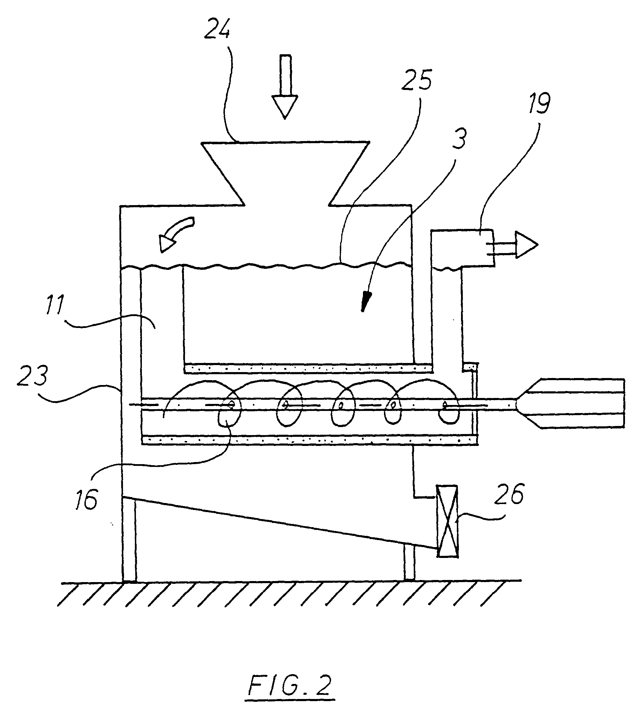

In Fig. 2 ist ein Vorlagetank 23 für flüssige, fetthaltige Kosubstrate dargestellt, die über einen Einfülltrichter 24 in den Vorlagetank 23 eingefüllt werden. Im Vorlagetank 23 ist eine ähnliche Vorbehandlungsvorrichtung 3, wie in Fig. 1 dargestellt, eingesetzt, jedoch ohne äußere Wärmedämmung 20. Bei der Anordnung in Fig. 2 wird damit erreicht, dass die heiße Vorbehandlungsvorrichtung bzw. das beheizte Vorbehandlungsrohr 10 Wärme an das in den Vorlagetank 23 eingefüllte Kosubstrat abgibt, wodurch das Kosubstrat ggf. in Verbindung mit einer (nicht dargestellten) Zusatzheizung flüssig gehalten wird. Der Einlauf in das Zuführrohr 11 liegt hier in der Höhe eines Flüssigkeitsspiegels 25 und entsprechend ist der Kosubstratauslass 19 als Überlauf in eine entsprechende Höhe nach oben geführt. An der tiefsten Stelle des Vorlagetanks 23 an einem schrägen Boden ist zudem ein Gesamtentleerungsschieber 26 angebracht.

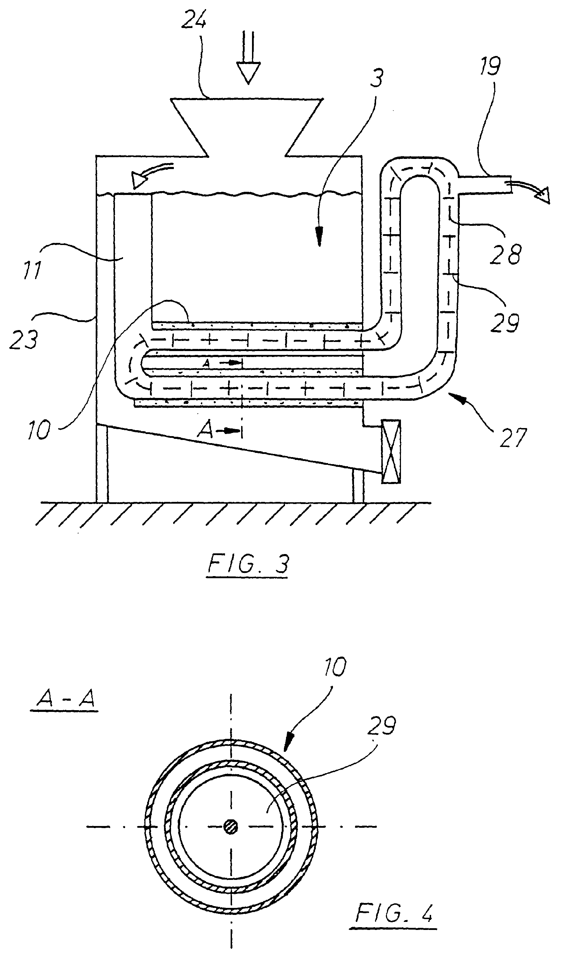

In Fig. 3 ist eine ähnliche Anordnung wie in Fig. 2 dargestellt mit einem Vorlagetank 23, einem Einfülltrichter 24 und einer Vorbehandlungsvorrichtung 3, die jedoch hinsichtlich ihrer Fördereinrichtung eine zu einer Schneckenförderung alternative Rohrfördereinrichtung 27 aufweist. Diese Rohrfördereinrichtung 27 läuft in einem ringförmigen Vorbehandlungsrohr 10, wobei Rohrteile mit einem Bogen aus dem Vorlagetank 23 zum Kosubstratauslass 19 geführt sind. Die Rohrfördereinrichtung 27 besteht dabei aus einem Zugelement 28 in der Art einer Kette oder eines Seils, woran im Abstand Förderelemente 29 angeordnet sind. Aus Fig. 4 ist ersichtlich, dass diese Förderelemente 29 scheibenförmig ausgeführt sind und den Innenrohrdurchmesser des doppelwandigen Vorbehandlungsrohres 10 weitgehend ausfüllen. Mittels dieser Förderelemente 29 wird Kosubstrat aus dem unteren Bereich des Zuführrohrs 11 aufgenommen und im Vorbehandlungsrohr 10 weiter transportiert.

## Patentansprüche

1. Kofermentationsanlage für die gemeinsame Vergärung eines Basissubstrats, vorzugsweise von Gülle, und organischer Rückstände aus Gewerbe, Industrie und Kommunen als Kosubstrate zur Erzeugung von Biogas in einem Nassvergärungsverfahren,
mit einem Fermenter als Reaktor mit eingebauter Mischeinrichtung,
mit einer Basissubstratzuführung zum Reaktor,
mit einer Kosubstratzuführung zum Reaktor,
mit einer Vorbehandlungsvorrichtung zur Zerkleinerung und zur Erwärmung von Kosubstraten, und
mit einem dem Reaktor nachgeschalteten Gasspeicher und einer Gasverwertungsvorrichtung insbesondere als Brenner oder Blockheizkraftwerk,
**dadurch gekennzeichnet,**
**dass** die Vorbehandlungsvorrichtung (3) wenigstens ein Vorbehandlungsrohr (10) aufweist,
**dass** das Vorbehandlungsrohr (10) an einem Ende für eine Kosubstrateinspeisung (18) hergerichtet ist und das andere Ende über einen Kosubstratauslass (19) mit dem Reaktor (2) direkt oder indirekt verbunden ist,
**dass** das Vorbehandlungsrohr (10) zumindest in Teilbereichen doppelwandig ausgeführt ist und das dadurch geschaffene Zwischenvolumen (13) an eine Heizwasserversorgung angeschlossen ist, so dass bei eingebrachtem Heizwasser (14) das Innenvolumen des Vorbehandlungsrohrs (10) erwärmbar und darin eingebrachte Kosubstrate thermisch vorbehandelbar, insbesondere thermisch hygienisierbar sind, und
**dass** im Vorbehandlungsrohr (10) eine sich axial erstreckende, antreibbare Fördereinrichtung (16; 27) als Transporteinrichtung und/oder als Misch- und/oder Zerkleinerungseinrichtung angebracht ist.

2. Kofermentationsanlage nach Anspruch 1, **dadurch gekennzeichnet, dass** an einem Ende des Vorbehandlungsrohrs (10) zur Kosubstrateinspeisung (18) ein etwa vertikales Zuführrohr (11) angeordnet ist dessen unterer Bereich eine Förderverbindung zum zugeordneten Ende der Fördereinrichtung (16; 27) aufweist, so dass von der angetriebenen Fördereinrichtung (16; 27) kontinuierlich Kosubstrat aus dem Zuführrohr (11) in das Vorbehandlungsrohr (10) gefördert wird und/oder dass am unteren Zuführrohrbereich eine Schließeinrichtung vorzugsweise als steuerbare Klappe oder steuerbarer Schieber (12) für eine chargenweise Beschickung des Vorbehandlungsrohrs (10) mit Kosubstrat vorgesehen ist.

3. Kofermentationsanlage nach Anspruch 1 oder Anspruch 2, **dadurch gekennzeichnet, dass** das Vorbehandlungsrohr (10) im wesentlichen horizontal gegebenenfalls mit einem leichten Anstieg von der Kosubstrateinspeisung (18) zum Kosubstratauslass (19) angeordnet ist.

4. Kofermentationsanlage nach einem der Ansprüche 1 bis 3, **dadurch gekennzeichnet, dass** der Kosubstratauslass (19) des Vorbehandlungsrohrs (10) im Bereich der Basissubstratzuführung (4) am Reaktor (2) angeordnet ist, für eine kontinuierliche Kosubstratzuführung zum Reaktor (2) und/oder dass am Kosubstratauslass (19) eine Schließeinrichtung vorzugsweise als steuerbare Klappe oder steuerbarer Schieber für eine chargenweise Kosubstratzuführung zum Reaktor (2) vorgesehen ist.

5. Kofermentationsanlage nach einem der Ansprüche 1 bis 4, **dadurch gekennzeichnet, dass** die Fördereinrichtung eine Schnecke (16) ist, die im Vorbehandlungsrohr (10) angeordnet ist.

6. Kofermentationsanlage nach Anspruch 5, **dadurch gekennzeichnet, dass** der Schneckenantrieb koaxial zur Schnecke (16) außerhalb des Vorbehandlungsrohrs (10) angeordnet ist.

7. Kofermentationsanlage nach Anspruch 5 oder 6, **dadurch gekennzeichnet, dass** die Schnecke (16) umfangsseitig strukturiert ist und/oder dass an der Innenwand des Vorbehandlungsrohrs (10) eine zugeordnete Struktur angebracht ist.

8. Kofermentationsanlage nach einem der Ansprüche 1 bis 4, **dadurch gekennzeichnet, dass** die Fördereinrichtung eine in wenigstens einem Vorbehandlungsrohr, vorzugsweise in einem ringförmigen Vorbehandlungsrohr umlaufende und antreibbare Rohrfördereinrichtung (27) ist, bei der an wenigstens einem Zugelement (28), vorzugsweise einer Kette und/oder einem Seil im Abstand angeordnete Förderelemente (29), vorzugsweise den Rohrdurchmesser weitgehend abdeckende Scheiben oder Schaufeln, angeordnet sind.

9. Kofermentationsanlage nach einem der Ansprüche 1 bis 8, **dadurch gekennzeichnet, dass** an der Außenwandung des Vorbehandlungsrohrs (10) eine Wärmedämmung (20) angebracht ist.

10. Kofermentationsanlage nach einem der Ansprüche 1 bis 9, **dadurch gekennzeichnet, dass** das Heizwasser (14) mit bestimmter Temperatur in einem Heizwasserkreislauf durch das Zwischenvolumen (13) gegebenenfalls über Leitsysteme dergestalt pumpbar ist, dass Kosubstrate im Vorbehandlungsrohr (10) auf wenigstens 70° C erhitzbar sind.

11. Kofermentationsanlage nach einem der Ansprüche 1 bis 10, **dadurch gekennzeichnet, dass** die Erhitzung des Heizwassers (14) direkt oder indirekt mittels Verbrennung von erzeugtem Biogas erfolgt.

12. Kofermentationsanlage nach einem der Ansprüche 1 bis 11, **dadurch gekennzeichnet, dass** mittels einer Steuereinrichtung die Fördergeschwindigkeit im Vorbehandlungsrohr (10) und/oder die wenigstens eine Schließeinrichtung (12) so steuerbar sind, dass die Verweildauer von Kosubstrat im Vorbehandlungsrohr (10) bei einer wenigstens 70° Celsius-Erhitzung wenigstens eine Stunde beträgt.

13. Kofermentationsanlage nach einem der Ansprüche 1 bis 12, **dadurch gekennzeichnet, dass** ein beheizbarer Vorlagetank (23) für flüssige, fetthaltige Kosubstrate vorgesehen ist und zumindest ein Teilbereich des beheizbaren Vorbehandlungsrohrs (10) im Vorlagetank (23) angeordnet ist.

14. Kofermentationsanlage nach Anspruch 13, **dadurch gekennzeichnet, dass** der Einlauf in das Zuführrohr (11) in der Höhe eines Flüssigkeitsspiegels (25) des Vorlagetanks (23) liegt und der Kosubstratauslass (19) als Überlauf in eine entsprechende Höhe nach oben geführt ist.
